# FASCICULE DE BREVET EUROPEEN

(11) **EP 3 134 533 B1**
(45) Date de publication et mention de la délivrance du brevet: **11.12.2019**
(21) Numéro de dépôt: 15725765.0
(22) Date de dépôt: 20.04.2015
(51) Int. Cl.: C12P 5/02, C12P 39/00, C12N 1/20

(54) **PROCÉDÉ DE PRODUCTION DE MÉTHANE PAR CO-CULTURE AÉROBIE DE MICROORGANISMES ANAÉROBIES**
VERFAHREN ZUR HERSTELLUNG VON METHAN MITTELS AEROBER COKULTUR VON ANAEROBEN MIKROORGANISMEN
METHOD FOR PRODUCING METHANE BY MEANS OF AEROBIC CO-CULTURE OF ANAEROBIC MICRO-ORGANISMS

(30) Priorité: 23.04.2014 FR 1453652; 20.06.2014 FR 1455745
(43) Date de publication de la demande: 01.03.2017
(73) Titulaire: Fondation Méditerranée Infection, 13005 Marseille (FR); Université d'Aix Marseille, 13284 Marseille Cedex 07 (FR); Centre National de la Recherche Scientifique (C.N.R.S.), 75016 Paris (FR); INSERM - Institut National de la Santé et de la Recherche Médicale, 75013 Paris (FR)
(72) Inventeur: RAOULT, Didier, F-13008 Marseille (FR); KHELAIFIA, Saber, F-13009 Marseille (FR); DRANCOURT, Michel, F-13012 Marseille (FR)
(74) Mandataire: Cabinet Beau de Loménie
(86) Numéro de dépôt international: PCT/FR2015/051067
(87) Numéro de publication internationale: WO 2015/162366

(56) Documents cités:
- WO-A1-2013/110891
- DE-A1-102006 035 213
- GB-A- 2 107 735
- M. J. LEE ET AL: "Enhanced bio-energy recovery in a two-stage hydrogen/methane fermentation process", WATER SCIENCE & TECHNOLOGY, vol. 59, no. 11, 1 juin 2009 (2009-06-01), page 2137, XP055092763, ISSN: 0273-1223, DOI: 10.2166/wst.2009.236
- MARK PIMENTEL ET AL: "Methanogens in Human Health and Disease", THE AMERICAN JOURNAL OF GASTROENTEROLOGY SUPPLEMENTS, vol. 1, no. 1, 1 juillet 2012 (2012-07-01) , pages 28-33, XP055159007, ISSN: 1948-9498, DOI: 10.1038/ajgsup.2012.6
- SHAN ZHANG ET AL: "Effects of VFAs Concentration on Bio-hydrogen Production with Clostridium Bifermentans 3AT-ma", ENERGY PROCEDIA, vol. 14, 8 mars 2012 (2012-03-08), pages 518-523, XP028466694, ISSN: 1876-6102, DOI: 10.1016/J.EGYPRO.2011.12.968 [extrait le 2012-03-08]
- BECKER P M ET AL: "Effects of plant antioxidants and natural vicinal diketones on methane production, studiedwith rumen fluid and a polylactate as maintenance substrate", ANIMAL FEED SCIENCE AND TECHNOLOGY, ELSEVIER, AMSTERDAM, NL, vol. 170, no. 3, 21 septembre 2011 (2011-09-21), pages 201-208, XP028107411, ISSN: 0377-8401, DOI: 10.1016/J.ANIFEEDSCI.2011.09.010 [extrait le 2011-10-06]
- N R KRIEG ET AL: "Microaerophily and Oxygen Toxicity", ANNUAL REVIEW OF MICROBIOLOGY, vol. 40, no. 1, 1 octobre 1986 (1986-10-01), pages 107-130, XP055069555, ISSN: 0066-4227, DOI: 10.1146/annurev.mi.40.100186.000543

## Description

La présente invention concerne un procédé de production de gaz méthane (CH4) par culture microbienne en conditions aérobie de microorganismes anaérobies.

Le méthane produit biologiquement a été considéré jusqu'à un passé récent comme n'étant produit que de façon anaérobie, c'est-à-dire sans oxygène, et usuellement en présence d'hydrogène, par fermentation anaérobie d'un certain nombre de déchets organiques.

Dans FR2537992, on produit du gaz CH4 par fermentation et dégradation anaérobie de déchets organiques. Pour ce faire, on injecte de l'hydrogène qui réagit avec le CO2 produit pour former du CH4 et ainsi diminuer le taux de CO2 en mélange avec CH4.il faut 10 à 5000 litres de gaz H2 par m3 de CH4 produit.

Dans FR2601690, on utilise un méthanogène thermophile *Methanococcus thermolitotrophicus* sous alimentation en H2/CO2, la culture se faisant dans un milieu contenant essentiellement une source d'azote et une source de sels assimilables à 110°C en anaérobie.

Des travaux récents, depuis 2006 (1), ont montré que dans la mer il était possible de détecter du méthane dans un milieu aérobie, exclusivement après enrichissement par du methylphosphonate, par un ou plusieurs microorganismes fixant l'azote (Karl DM, Beversdorf L, Björkman KM, Church MJ, Martinez A, DeLong EF. Aerobic production of methane in the sea. Nat Geosci 2008;1:473-8).

Dans GB 2 107 735, on décrit un procédé de fermentation pour la production de gaz méthane comprenant la co-culture d'une souche Méthanobacterium et d'une bactérie *Clostridium bifermentans* dans des conditions anaérobies sous une atmosphère contenant de l'hydrogène et du dioxide de carbone (CO₂).

Par ailleurs, le méthane naturel est produit dans l'intestin en conditions anaérobies sous l'activité de microorganismes méthanogènes. Dans le tube digestif le méthane est produit sous l'action de microorganismes Archae méthanogènes appelés, en particulier *Methanobrevibacter smithii ou Methanomassilicoccus luminyensis,* qui fabriquent du méthane à partir de molécules d'hydrogène (H2) produites par la fermentation des sucres par des bactéries anaérobies du tube digestif, en particulier par les Bacteroides, en particulier par *Bacteroides thetaiotaomicron.*

Le but de la présente invention est de fournir un nouveau procédé biotechnologique de production de méthane en conditions aérobies.

Les inventeurs ont découvert de façon surprenante qu'il était possible de permettre la culture de bactéries anaérobies en associant au milieu de culture, un composé anti-oxydant tout en maintenant la culture d'archaea méthanogène.

Plus précisément, les inventeurs ont observé que l'addition de composés antioxydants dans un milieu de culture en atmosphère aérobie avec une bactérie anaérobie apte à produire de l'hydrogène, permettait aussi la culture d'une archaea méthanogène, du fait de la production d'hydrogène par ladite bactérie anaérobie. Ils ont pu mesurer la production d'hydrogène en présence de ladite bactérie anaérobie et la production de méthane par ladite archaea, faite de façon aérobie, par l'association des deux microorganismes anaérobies, notamment *Methanobrevibacter smithii* ou *Methanomassiliicoccus luminyensis* et *Bacteroides thetaiotaomicron.* Ainsi, la réalisation de chambres de co-culture avec d'une part *Bacteroides thetaiotaomicron,* ou d'une autre bactérie anaérobie, et d'autre part *Methanobrevibacter smithii* ou *Methanomassiliicoccus luminyensis,* ou un autre organisme méthanogène permet de fournir une quantité significative de méthane.

La présente invention consiste donc essentiellement dans la production biologique de méthane (Biogaz) par une association d'une bactérie méthanogène et une bactérie anaérobie cultivant en aérobie dans un milieu riche en composants antioxydants et contenant une source d'hydrates de carbone.

Pour ce faire, la présente invention fournit un procédé de production de gaz méthane dans un réacteur par co-culture en atmosphère aérobie, de préférence air ambiant d'au moins :
- un premier microorganisme consistant en une bactérie anaérobie apte à produire de l'hydrogène par fermentation en présence d'un substrat et /ou d'un milieu de culture comprenant ou complémenté en hydrate(s) de carbone, notamment de l'amidon et/ou des sucres, et
- un deuxième microorganisme consistant en une Archaea méthanogène apte à produire du méthane à partir d'hydrogène et d'un substrat et /ou milieu de culture comprenant ou complémenté en hydrate(s) de carbone, notamment de l'amidon et/ou des sucres, et
- un substrat organique et minéral comprenant des composants de milieux de culture aptes à permettre la culture des deux dits premier et deuxième microorganismes anaérobies, ledit milieu de culture comprenant ou étant complémenté en hydrate(s) de carbone, notamment de l'amidon et/ou des sucres, et étant en outre supplémenté en composé(s) anti-oxydant(s).

Plus particulièrement, les autres conditions de culture, notamment de température, sont les conditions appropriées pour la culture desdits microorganismes. En particulier, on doit augmenter la température pour incuber si nécessaire à une température favorisant la croissance desdits microorganismes, notamment à au moins 30°C voire 37°C.

On comprend que le procédé selon la présente invention ne nécessite pas l'apport d'hydrogène externe et que le réacteur de co-culture comprend des moyens de récupération du gaz méthane produit.

De préférence, on réalise tout d'abord la culture dudit premier microorganisme dans ledit substrat et on introduit ledit deuxième microorganisme après que ledit premier microorganisme a déjà produit des produits de fermentation et de l'hydrogène.

On entend ici par « composé antioxydant », un composé à propriété antioxydante, c'est-à-dire qu'il diminue ou empêche l'oxydation d'autres substances, notamment en l'espèce d'autres substances impliquées dans le procédé de l'invention dans les conditions du procédé selon l'invention.

Plus particulièrement, ledit composé antioxydant est choisi de préférence parmi l'acide ascorbique, l'acide urique et le glutathion (γ-L-Glutamyl-L-cysteinylglycine). L'acide ascorbique et l'acide urique sont préférés car ils sont capables à des doses précises de permettre la culture à un niveau d'oxygène plus élevé.

Plus particulièrement encore, ledit composé antioxydant est mis en œuvre à une concentration de 1 µg/ml à 2 mg/ml, ou concentration molaire de 10⁻⁶ M à 10⁻² M, de préférence au moins 1g/l.

De préférence, ledit milieu comprend une substance tampon régulateur de pH pour ajuster le pH de 7 à 7,5.

Plus particulièrement, ladite Archaea est une Archaea choisie parmi les Archae suivantes: *Méthanobrevibacter, Methanosphaera, Methanomassiliicoccus, Methanobacterium, Methanococcus et Methanosaeta.*

Plus particulièrement, ladite Archaea est une Archaea choisie parmi les Archae suivantes : *Methanobrevibacter smithii, Methanobrevibacter oralis, Methanosphaera stadtmanae, Methanomassiliicoccus luminyensis, Methanobacterium beijingense et Methanosaeta concilii.*

Plus particulièrement encore, ladite Archea est *Méthanobrevibactera smithii ou Methanomassiliicoccus luminyensis.*

Les bactéries anaérobies strictes c'est-à-dire qu'elles ne sont pas capables de cultiver en présence d'oxygène ou dans des concentrations inférieures à 1%, le plus communément inférieure à 0.1%, idéalement 0%. Parmi les bactéries anaérobies strictes, on cite plus particulièrement les bactéries extracellulaires, c'est à dire des bactéries qui ne peuvent vivre qu'à l'extérieur de cellules.

Plus particulièrement, ladite bactérie anaérobie apte à produire de l'hydrogène est choisie parmi les bactéries des familles *Actinobacteria* et *Bacteroidetes.*

De préférence, ladite bactérie anaérobie est du genre *Bacteroides,* Ces bactéries sont connues en particulier pour digérer l'amidon en produisant de l'hydrogène.

Plus particulièrement, ladite bactérie anaérobie est *Bacteroides thetaiotaomicron.*

On cite aussi comme *Actinobacteria,* la bactérie *Lactococcus lactis.*

Plus particulièrement, ledit milieu de culture comprend les composants que l'on retrouve dans les milieux de base de culture aptes à cultiver une archaea ou une bactérie anaérobie, comprenant au moins :
- plusieurs sources de carbone,
- une source de phosphore, de préférence un sel de phosphate,
- une source d'azote, de préférence un sel d'ammonium,
- au moins un sel de métaux choisi parmi K, Mg, Na, Ca, de préférence NaCl.

Plus particulièrement, ledit milieu culture est un milieu acellulaire est choisi parmi un milieu axénique constitué de substances chimiques ou biologiques défini qualitativement et quantitativement, et un milieu acellulaire comprenant un extrait de broyat ou lysat de tissu pluricellulaire.

Plus particulièrement, ledit milieu de culture est un milieu acellulaire conventionnel de bactérie anaérobie, de préférence un milieu comprenant des composant choisis parmi un extrait de broyat ou lysat de tissu pluricellulaire, un digestat enzymatique, notamment un digestat enzymatique de caséine, soja et/ou de tissu animal, une peptone, un extrait de levure, un sucre tel que dextrose ou glucose, un sel NaCl et/ou Na₂PO₄.

Plus particulièrement encore, ledit milieu de culture est un milieu conventionnel de culture de bactéries anaérobies tel que les milieux dits bouillon de type cœur-cervelle, milieux Columbia à 5% de sang de mouton ou milieu Schaedler tels que décrits ci-après. D'autres milieux conventionnels appropriés sont les milieux Brucella ou Wilkins-Chagren. De tels milieux de culture acellulaires sont bien connus de l'homme de l'art.

On peut utiliser en particulier des milieux de culture polyvalents pour microorganismes anaérobies, notamment le milieu de Schaedler, ledit milieu étant complémenté en hydrate(s) de carbone, de préférence de l'amidon et de(s) sucre(s), et en dit(s) composé(s) antioxydant(s).

Les inventeurs ont en effet testé différentes molécules présentant une activité antioxydante et ont découvert que certains composés anti-oxydants dans certaines concentrations présentent un effet supérieur sur la croissance des dites bactéries.

D'autres caractéristiques et avantages de la présente invention ressortiront mieux à la lecture de la description qui va suivre, faite de manière illustrative et non limitative, d'un exemple de réalisation.

Pour illustrer l'invention, les inventeurs ont cultivé des archaea méthanogènes réputées anaérobies strictes (réputées ne cultiver qu'en l'absence stricte d'oxygène), en atmosphère aérobie (c'est à dire à l'air libre, contenant environ 16% d'oxygène) et en présence de bactéries réputées anaérobie stricte, dans des milieux de culture de bactéries et d'archae réputées anaérobies, lesdits milieux de culture étant complémentés en composés anti-oxydants pour supporter la croissance en atmosphère d'air ambiant et complémentés en hydrate(s) de carbone pour produite H2 et CH4.

### 1) Souches utilisées.

Une archaea méthanogène *Methanobrevibacter smithii* souche DSM 861 a été obtenue auprès de la collection allemande de microorganismes et de cultures cellulaires (DSMZ, Braunschweig, Allemagne) aussi déposée à l'ATCC sous le numéro ATCC 35061.

Une archaea *Methanomassiliicoccus luminyensis* déposée à la collection de dépôt de microorganismes DSMZ (Allemagne), conformément au Traité de Budapest, sous le numéro DSM 24529 a aussi été testée décrite dans FR124779 (publié sous le numéro 2 990954).

Par ailleurs, une souche de bactérie anaérobie *Bacteroides thetaiotaomicron* a été obtenue au travers de l'étude "culturomics" des inventeurs (Lagier JC et al., Microbial culturomics: paradigm shift in the human gut microbiome study. Clin Microbiol Infect. 2012;18:1185-93) aussi accessible dans divers collections de dépôt (CSUR P766 aussi déposée selon le Traité de Budapest à la collection de dépôt de micro-organismes DSMZ (Allemagne) le 19 mai 2014 sous le numéro DSM 28808, d'autres souches sont aussi accessibles dans divers collections de dépôt telles que les souches DSM 2079, ATCC 29148 et NCTC 10582).

Une autre souche de bactérie anaérobie suivante a aussi été testée : *Lactococcus lactis* déposée selon le Traité de Budapest à la collection de dépôt de micro-organismes DSMZ (Allemagne) le 19 mai 2014 sous le numéro DSM 28809, aussi disponible dans divers collections de dépôt dont la Collection Nationale de cultures de micro-organismes CNCM 1-2716.

### 2) Culture aérobie des microorganismes anaérobies à l'aide d'antioxydants.

Pour leur production en quantité suffisante, les deux souches M. *smithii* ou *M. luminyensis* et *B. thetaiotaomicron* ont été cultivées en atmosphère anaérobie à 37 °C dans un milieu de culture polyvalent. On a testé le milieu de Schaedler (Reference 42098 ; BioMérieux, La Balmes-les-Grottes, France) ainsi que le milieu dénommé «milieu SAB » décrit dans FR 124779 (publié sous le numéro 2 990954) et WO 2013/0044933 habituellement utilisé pour cultiver les archaea méthanogènes humains et qui apparait approprié aussi pour la culture de bactéries anaérobies.

Le milieu Schaedler (commercialisé par Biomerieux, Marcy l'étoile, France) présentait la composition suivante pour 1 litre :

| | |
|---|---|
| - Digestat enzymatique de caséine | 5.6 g |
| - Digestat enzymatique de tourteau de soja | 1 g |
| - Digestat enzymatique de tissus animal | 5 g |
| - Extrait de levure | 5 g |
| - NaCl | 1.7 g |
| - Phosphate de potassium | 0.82 g |
| - Dextrose | 5.82 g |
| - Tris (hydroxymethyl) Aminomethane | 3 g |
| - Hemine | 0.01 g |
| - L-cysteine | 0.4g |

Le milieu SAB utilisé était dépourvu de Na2S et de L-cystéine et comprenait : NiCl2x6H2O (0,07mg/l), Na2MoO4 x 2H2O (0,02mg/l), FeSO4 x 7H2O (0,2 mg/l), MgS04 x 7H2O (0,01 g/l), K2HP04 (0,5 g/l), KH2P04 (0,5 g/l), KCl (0,05 g/l), CaCl2 (0,05 g/l), NaCl (1,5 g/l), NH4Cl (1 g/l), Na-Acétate (1 g/l), Extrait de levure (1 g/l), Biotrypticase (1 g/l), Solution d'oligoéléments de Widdel (2 ml/l), Solution d'oligoéléments de Balch (10 ml/l), Résazurine (1 mg/l), Na2Se2O3.5H2O (0,015 mg/l), Na2WO4.2H20 (0,02 mg/l), NiCl2.6H2O (0,07 mg/l), NaHC03 (4 g/l), Na-Formate (0,4 g/l), Methanol (40 mM), Solution de vitamines de milieu de Balch (10 ml/l), Acide valérique (0,6 g/l), Acide isovalérique (0,6 g/l), Acide 2-méthylbutyrique (0,6 g/l), Acide isobutyrique (0,6 g/l), Acide 2-méthylvalérique (0,6 g/l).

Les oligoéléments de Balch comprennent NaCl, FeS04, ZnSO4, MgS04, MnS04, CoS04, H3BO3, NiCl2, Na2MoO4, CaCl2.

Les oligoéléments de Widdel comprennent FeCl2, CoCl2, MnCl2, ZnCl2, H3B03, Na2MoO4, NiCl2 et CuCl2.

Les vitamines du milieu de Balch comprennent la biotine, acide folique, hydrochlorure de pyridoxine, hydrochlorure de thiamine, riboflavine, acide nicotinique, pantothénate de DL-calcium, vitamine B12, Acide p-aminobenzoïque et acide lipoique.

Ce milieu Schaedler ainsi que ce milieu SAB ont été complémentés par l'ajout des hydrate(s) de carbone à savoir de 1 g/L d'amidon de riz et de 1 g/L de glucose (Sigma-Aldrich, Saint-Quentin Fallavier, France) et l'ajout de composés anti-oxydants à savoir complémenté par l'ajout de 1 g/L d'acide ascorbique (VWR International, Louvain, Belgique), 0,1 g/L d'acide urique et 0,1 g/L de glutathion (Sigma-Aldrich, Saint-Quentin Fallavier, France).

La résazurine est mise en oeuvre comme un indicateur d'oxydoréduction à une concentration de 0,1mg/mL pour contrôler la présence d'oxygène (la résazurine oxydée a une couleur rose, et devient transparente en l'absence d'oxygène).

La culture aérobie en air ambiant de *M. smithii* ou *M. luminyensis* et *B. thetaiotaomicron* a été effectuée dans des récipients séparés et dans un même récipient incubés à 37°C contenant le milieu de culture complémenté par l'ajout de composés anti-oxydants et composés source de carbone. Le pH a été ajusté à 7,5 par ajout de KOH 10M.

Les deux souches ont été cultivées séparément ainsi qu'en co-culture, en condition aérobie et par l'inoculation de 10⁵ organismes/mL de chaque souche avec le milieu de culture complémenté selon la présente invention et parallèlement avec le milieu Schaedler ou SAB complémenté par les hydrates de carbone mentionnés ci-dessus mais en revanche sans composés anti-oxydants.

Les témoins positifs consistent en un tube contenant le milieu de culture complémenté ci-dessus, inoculé en conditions anaérobie par 10⁸ microorganismes/L de *M. smithii* ou *M. luminyensis* en présence du mélange gazeux de 80% H2 + 20% de CO2 à la pression de deux atmosphères requise pour la croissance optimale des archaea méthanogènes. Le milieu de culture complémenté inoculé en anaérobie par 10⁸ microorganismes/L de *M. smithii* ou *M. luminyensis* sans ce mélange de gaz a été introduit pour vérifier la croissance de cette archaea méthanogène sans H2. Le milieu de culture supplémenté en ajoutant 1 g/L d'amidon de riz et de 1 g/L de glucose inoculé en anaérobie par 10⁸ cellules/L de *B. thetaiotaomicron* a été introduit comme témoin positif et pour vérifier la production de H2 par B. *thetaiotaomicron* en culture anaérobie. Ces contrôles ont été effectués en parallèle en atmosphère ambiante (aérobie). Le milieu de culture non-inoculé a été introduit comme contrôle négatif.

### 3) Détection de la croissance par chromatographie en phase gazeuse.

La croissance de *M. smithii* ou *M. luminyensis* a été évaluée quotidiennement par la production de méthane et la croissance de B. *thetaiotaomicron* a été évaluée quotidiennement par la production d'hydrogène. La mesure de méthane et d'hydrogène a été réalisée l'aide d'un chromatographe en phase gazeuse GC-8A (Shimadzu, Champs-sur-Marne, France) équipé d'un détecteur de conductivité thermique et une Chromosorb WAW 80/100 mailles colonne SP100 (Alltech, Carquefou, France). L'azote N2 à une pression de 100 kPa a été utilisé comme gaz porteur. Le détecteur et les températures de l'injecteur étaient de 200°C et la température de la colonne était de 150°C.

### 4) Résultats.

### 4.1) Contrôles.

Les contrôles négatifs sont restés négatifs sans croissance survenant après une semaine d'incubation indiquant que les résultats rapportés ici ne sont pas simplement le résultat d'une contamination par d'autres microorganismes.

Les contrôles positifs étaient positifs avec une production de méthane observée dans la culture anaérobie de *M. stmithii* ou *M. luminyensis* et avec une production d'hydrogène observée dans la culture anaérobie *B. thetaiotaomicron.* Il n'y a eu aucune production de méthane décelable dans la culture de *M. smithii* ou *M. luminyensis* inoculée seul en anaérobie et en aérobie sans mélange de gaz. Egalement, la culture de *B. thetaiotaomicron* inoculée en aérobie sans composés antioxydants est restée négative et l'hydrogène n'a pas été produit.

### 4.2) Co-culture aérobie.

Après une incubation de 24 heures à 37°C en air ambiant (dans des conditions aérobies), un milieu de culture sans composés anti-oxydants a conservé sa couleur rose indiquant la présence d'oxygène. Le milieu de culture aérobie avec les composés anti-oxydants est devenu transparent indiquant l'absence d'oxygène après sa réduction par les composés antioxydants.

Le milieu de culture inoculé en aérobie par *M. smithii* ou *M. luminyensis* avec les composés anti-oxydants est devenu transparent, mais la culture est restée négative et le méthane n'a pas été produit. Le milieu de culture inoculé en aérobie par *B. thetaiotaomicron* en présence des antioxydants est devenu transparent et une croissance bactérienne a été observée avec production d'hydrogène.

Les co-cultures de *M. smithii* ou *M. luminyensis* et *B. thetaiotaomicron* réalisées dans des conditions aérobies avec des composés anti-oxydants donnaient toutes une culture positive pour *B*. *thetaiotaomicron* avec production d'hydrogène après une incubation de 24 heures. En revanche, dans certaines expériences, aucune croissance n'était observée pour *M. smithii* ou *M. luminyensis* (bien que le milieu de culture soit devenu transparent). Les inventeurs ont émis l'hypothèse que *M. smithii* ou *M. luminyensis* est mort en raison de son exposition à l'oxygène avant que le milieu n'ait été réduit par les composés anti-oxydants.

Des expériences ont été effectuées avec introduction de *B. thetaiotaomicron* à t0 et *M. smithii* ou *M. luminyensis* ajoutée à t0+ 24 heures d'incubation. L'addition de *M. smithii* ou *M. luminyensis* après 24 heures d'incubation a permis dans tous les cas de rétablir sa croissance par la consommation de l'hydrogène préalablement produit par *B*. *thetaiotaomicron* en présence de composés antioxydants et en lui évitant une exposition mortelle à l'oxygène.

Cette même expérience a été réalisée sans composé anti-oxydant et la culture est restée négative pour les deux souches testées.

D'autre part, la bactérie anaérobie *Lactococcus lactis* DSM 28809 a aussi été testée avec succès combinée à ces deux archaea.

### 4.3) Interprétation.

Ces résultats indiquent qu'il est possible de cultiver à l'air ambiant (condition aérobie) des bactéries réputées strictement anaérobies, dans un milieu approprié contenant un mélange approprié d'antioxydants.

Dans ces conditions, les bactéries anaérobies produisent de l'hydrogène qui peut être alors utilisé dans un deuxième temps par les archaea méthanogènes pour la production de méthane.

Il est démontré qu'il est possible de produire du méthane à l'air ambiant, dans des conditions appropriées de co-culture de bactéries et de méthanogènes anaérobies dans un milieu de culture complémenté en composés source de carbone et composés anti-oxydants.

L'introduction des archaea méthanogènes dans un milieu contenant déjà des produits de fermentation et de l'hydrogène est préférable.

## Revendications

1. Procédé de production de gaz méthane par co-culture dans un réacteur en atmosphère aérobie, de préférence air ambiant, d'au moins :
- un premier microorganisme consistant en une bactérie anaérobie apte à produire de l'hydrogène par fermentation en présence d'un substrat et /ou d'un milieu de culture comprenant ou complémenté en hydrate(s) de carbone, et
- un deuxième microorganisme consistant en un Archaea méthanogène apte à produire du méthane à partir d'hydrogène et d'un substrat et /ou milieu de culture comprenant ou complémenté en hydrate(s) de carbone, et
- un substrat organique et minéral comprenant des composants de milieux de culture aptes à permettre la culture des deux dits premier et deuxième microorganismes, ledit milieu de culture comprenant ou étant complémenté en hydrate(s) de carbone, et étant en outre supplémenté en composé(s) anti-oxydant(s).

2. Procédé selon la revendication 1, **caractérisé en ce qu'**on réalise tout d'abord la culture dudit premier microorganisme dans ledit substrat et on introduit ledit deuxième microorganisme après que ledit premier microorganisme a déjà produit des produits de fermentation et de l'hydrogène.

3. Procédé selon l'une des revendications 1 ou 2, **caractérisé en ce que** le(s) dit(s) composé(s) anti-oxydant (s) est (sont) choisi(s) parmi l'acide ascorbique, l'acide urique et le glutathion.

4. Procédé selon l'une des revendications 1 à 3, **caractérisé en ce que** ladite Archaea est une Archaea choisie parmi les archae suivantes: *Méthanobrevibacter, Methanosphaera, Methanomassiliicoccus, Methanobacterium, Methanococcus et Methanosaeta.*

5. Procédé selon la revendication 4, **caractérisé en ce que** ladite archaea est une archaea choisie parmi les archae suivantes: *Methanobrevibacter smithii, Methanobrevibacter oralis, Methanosphaera stadtmanae, Methanomassiliicoccus luminyensis, Methanobacterium beijingense et Methanosaeta concilii.*

6. Procédé selon la revendication 5, **caractérisé en ce que** ledit archea est *Méthanobrevibacter smithii ou Methanomassiliicoccus luminyensis.*

7. Procédé selon l'une des revendications 1 à 6, **caractérisé en ce que** ladite bactérie anaérobie apte à produire de l'hydrogène est choisie parmi les bactéries des familles *Bacteroidetes* et *Actinobacteria.*

8. Procédé selon la revendication 7, **caractérisé en ce que** ladite bactérie anaérobie est du genre *Bacteroides.*

9. Procédé selon la revendication 8, **caractérisé en ce que** ladite bactérie anaérobie est *Bacteroides thetaiotaomicron ou Lactococcus lactis.*

10. Procédé selon la revendication 7, **caractérisé en ce que** ladite bactérie anaérobie est *Lactococcus lactis.*

11. Procédé selon l'une des revendications 6 à 10, **caractérisé en ce que** ledit organisme est choisi parmi les souches de *Méthanobrevibacter smithii DSM 861 et la souche Methanomassiliicoccus luminyensis DSM 24529 et la bactérie anaérobie est choisie parmi la souche Bacteroides thetaiotaomicron DSM 28808 et la souche Lactococcus lactis DSM 28809.*

12. Procédé selon l'une des revendications 1 à 11, **caractérisé en ce que** ledit milieu de culture comprend au moins les composants suivants:
- plusieurs sources de carbone,
- une source de phosphore, de préférence un sel de phosphate,
- une source d'azote, de préférence un sel d'ammonium,
- au moins un sel de métaux choisi parmi K, Mg, Na, Ca, de préférence NaCl.

13. Procédé selon la revendication 12, **caractérisé en ce que** ledit composé antioxydant est mis en œuvre à une concentration de 1 mg/l à 2 g/l, de préférence au moins 1g/l.

14. Procédé selon l'une des revendications 1 à 13, **caractérisé en ce que** ledit milieu comprend une substance tampon régulateur de pH pour ajuster le pH de 7 à 7,5.

15. Procédé selon l'une des revendications 1 à 14, **caractérisé en ce que** ledit milieu comprend les composants du milieu de Schaedler complémenté en hydrate(s) de carbone, de préférence de l'amidon et de(s) sucre(s), et en dit(s) composé(s) antioxydant(s).

## Patentansprüche

1. Verfahren zur Produktion von Methangas durch Kokultur in einem Reaktor mit aerober Atmosphäre, vorzugsweise Umgebungsluft, von zumindest:
- einem ersten Mikroorganismus, der aus einem anaeroben Bakterium besteht, das dazu geeignet ist, durch Fermentation in Anwesenheit eines Substrats und/oder eines Kulturmediums, das Kohlenhydrat(e) umfasst oder damit komplementiert ist, Wasserstoff zu produzieren, und
- einem zweiten Mikroorganismus, der aus einem methanogenen Archaeon besteht, das dazu geeignet ist, ausgehend von Wasserstoff und einem Substrat und/oder Kulturmedium, das Kohlenhydrat(e) umfasst oder damit komplementiert ist, Methan zu produzieren, und
- einem organischen und mineralischen Substrat, das Kulturmedien-Komponenten umfasst, die dazu geeignet sind, die Kultur der beiden ersten und zweiten Mikroorganismen zu ermöglichen, wobei das Kulturmedium Kohlenhydrat(e) umfasst oder damit komplementiert ist und ferner durch (eine) antioxidative Verbindung(en) ergänzt ist.

2. Verfahren nach Anspruch 1, **dadurch gekennzeichnet, dass** zuerst die Kultur des ersten Mikroorganismus in dem Substrat durchgeführt wird und der zweite Mikroorganismus eingebracht wird, nachdem der erste Mikroorganismus bereits Fermentationsprodukte und Wasserstoff produziert hat.

3. Verfahren nach einem der Ansprüche 1 oder 2, **dadurch gekennzeichnet, dass** die antioxidative(n) Verbindung(en) aus Ascorbinsäure, Harnsäure und Glutathion ausgewählt ist/sind.

4. Verfahren nach einem der Ansprüche 1 bis 3, **dadurch gekennzeichnet, dass** das Archaeon ein aus den folgenden Archaea ausgewähltes Archaeon ist:
*Methanobrevibacter, Methanosphaera, Methanomassiliicoccus, Methanobacterium, Methanococcus* und *Methanosaeta.*

5. Verfahren nach Anspruch 4, **dadurch gekennzeichnet, dass** das Archaeon ein aus den folgenden Archaea ausgewähltes Archaeon ist: *Methanobrevibacter smithii, Methanobrevibacter oralis, Methanosphaera stadtmanae, Methanomassiliicoccus luminyensis, Methanobacterium beijingense* und *Methanosaeta concilii.*

6. Verfahren nach Anspruch 5, **dadurch gekennzeichnet, dass** das Archaeon *Methanobrevibacter smithii* oder *Methanomassiliicoccus luminyensis* ist.

7. Verfahren nach einem der Ansprüche 1 bis 6, **dadurch gekennzeichnet, dass** das anaerobe Bakterium, das dazu geeignet ist, Wasserstoff zu produzieren, aus den Bakterien der Familien *Bacteroidetes* und *Actinobacteria* ausgewählt ist.

8. Verfahren nach Anspruch 7, **dadurch gekennzeichnet, dass** das anaerobe Bakterium aus der Gattung *Bacteroides* stammt.

9. Verfahren nach Anspruch 8, **dadurch gekennzeichnet, dass** das anaerobe Bakterium *Bacteroides thetaiotaomicron* oder *Lactococcus lactis* ist.

10. Verfahren nach Anspruch 7, **dadurch gekennzeichnet, dass** das anaerobe Bakterium *Lactococcus lactis* ist.

11. Verfahren nach einem der Ansprüche 6 bis 10, **dadurch gekennzeichnet, dass** der Organismus aus den Stämmen von *Methanobrevibacter smithii* DSM und dem Stamm *Methanomassiliicoccus luminyensis* DSM 24529 ausgewählt ist und das anaerobe Bakterium aus dem Stamm *Bacteroides* thetaiotaomicron DSM 28808 und dem Stamm *Lactococcus lactis* DSM 28809 ausgewählt ist.

12. Verfahren nach einem der Ansprüche 1 bis 11, **dadurch gekennzeichnet, dass** das Kulturmedium zumindest die folgenden Komponenten umfasst:
- mehrere Kohlenstoffquellen,
- eine Phosphorquelle, vorzugsweise ein Phosphatsalz,
- eine Stickstoffquelle, vorzugsweise ein Ammoniumsalz,
- zumindest ein Salz eines Metalls, das aus K, Mg, Na, Ca ausgewählt ist, vorzugsweise NaCl.

13. Verfahren nach Anspruch 12, **dadurch gekennzeichnet, dass** die antioxidative Verbindung in einer Konzentration von 1 mg/l bis 2 g/l, vorzugsweise zumindest 1g/l, eingesetzt wird.

14. Verfahren nach einem der Ansprüche 1 bis 13, **dadurch gekennzeichnet, dass** das Medium eine pH-regelnde Puffersubstanz umfasst, um den pH auf 7 bis 7,5 einzustellen.

15. Verfahren nach einem der Ansprüche 1 bis 14, **dadurch gekennzeichnet, dass** das Medium die Komponenten des Schaedler-Mediums umfasst, komplementiert mit Kohlenhydrat(en), vorzugsweise Stärke und Zucker(n), und mit der/den antioxidativen Verbindung(en).

## Claims

1. Process for producing methane gas by co-culturing in a reactor in an aerobic atmosphere, preferably ambient air, at least:
- a first microorganism consisting of an anaerobic bacterium capable of producing hydrogen by fermentation in the presence of a substrate and/or a culture medium comprising or supplemented with carbohydrate(s), and
- a second microorganism consisting of a methanogenic Archaea capable of producing methane from hydrogen and a substrate and/or culture medium comprising or supplemented with carbohydrate(s), and
- an organic and mineral substrate comprising components of culture media capable of allowing the culture of both said first and second microorganisms, said culture medium comprising or being supplemented with carbohydrate(s), and being further supplemented with antioxidant compound(s).

2. Process according to claim 1, **characterised in that**, first, said first microorganism is cultured in said substrate and said second microorganism is introduced after said first microorganism has already produced fermentation products and hydrogen.

3. Process according to one of claims 1 or 2, **characterised in that** said antioxidant compound(s) is (are) selected from ascorbic acid, uric acid and glutathione.

4. Process according to one of claims 1 to 3, **characterised in that** said Archaea is an Archaea selected from the following archaea: *Methanobrevibacter, Methanosphaera, Methanomassitiicoccus, Methanobacterium, Methanococcus and Methanosaeta.*

5. Process according to claim 4, **characterised in that** said archaeon is an archaeon selected from the following archaea: *Methanobrevibacter smithii, Methanobrevibacter oralis, Methanosphaera stadtmanae, Methanomassiliicoccus iuminyensis, Methanobacterium beijingense and Methanosaeta concilii.*

6. Process according to claim 5, **characterised in that** said archaeon is *Methanobrevibacter smithii or Methanomassiliicoccus luminyensis.*

7. Process according to one of claims 1 to 6, **characterised in that** said anaerobic bacteria capable of producing hydrogen is selected from the bacteria of the families *Bacteroidetes* and *Actinobacteria.*

8. Process according to claim 7, **characterised in that** said anaerobic bacterium is of the genus *Bacteroides.*

9. Process according to claim 8, **characterised in that** said anaerobic bacterium is *Bacteroides thetaiotaomicron or Lactococcus lactis.*

10. Process according to claim 7, **characterised in that** said anaerobic bacterium is *Lactococcus lactis.*

11. Process according to one of claims 6 to 10, **characterised in that** said organism is selected from the strains of *Methanobrevibacter smithii DSM and the strain Methanomassiliicoccus luminyensis DSM 24529 and the anaerobic bacteria is selected from the strain Bacteroides thetaiotaomicron DSM 28808 and the strain Lactococcus lactis DSM 28809.*

12. Process according to one of claims 1 to 11, **characterised in that** said culture medium comprises at least the following components:
- several sources of carbon,
- a source of phosphorus, preferably a phosphate salt,
- a source of nitrogen, preferably an ammonium salt,
- at least one metal salt selected from K, Mg, Na, Ca, preferably NaCl.

13. Process according to claim 12, **characterised in that** said antioxidant compound is used at a concentration of 1 mg/l to 2 g/l, preferably at least 1 g/l.

14. Process according to one of claims 1 to 13, **characterised in that** said medium comprises a pH-controlling buffer substance for adjusting the pH from 7 to 7.5.

15. Process according to one of claims 1 to 14, **characterised in that** said medium comprises the components of the Schaedler medium supplemented with carbohydrate(s), preferably starch and sugar(s), and said antioxidant compound(s).
